# EUROPÄISCHE PATENTANMELDUNG

(11) **EP 2 253 274 A1**
(43) Veröffentlichungstag der Anmeldung: **24.11.2010**
(21) Anmeldenummer: 10004023.7
(22) Anmeldetag: 16.04.2010
(51) Int. Cl.: A61B 8/08

(54) **Vorrichtung für eine Ultraschallgestützte Computertomographie mit erweitertem Messbereich**

(30) Priorität: 20.05.2009 DE 102009022060
(71) Anmelder: Karlsruher Institut für Technologie, 76131 Karlsruhe (DE)
(72) Erfinder: Gumb, Lothar, 76676 Graben-Neudorf (DE); Vagner, Jörg, 76344 Eggenstein-Leopoldshafen (DE); Ruiter, Nicole, 76131 Karlsruhe (DE)
(74) Vertreter: Gärtner, Stephan

(57) **Zusammenfassung**

Vorrichtung für eine Ultraschallgestützte Computertomographie an Objekten, umfassend einen mit einem Ankopplungsmedium gefüllten Behälter (1) mit einer Behälteröffnung, eine Anzahl von Ultraschallwandlern (2), die in den Behälter auf das Ankopplungsmedium einwirken sowie eine Adapterauflage (5) an der Behälteröffnung als Dichtelement zwischen Objekt und Behälteröffnung. Die Aufgabe liegt darin, eine Vorrichtung der genannten Art mit einem erweiterten Messbereich vorzuschlagen, die insbesondere eine zuverlässige Mammographie der gesamten Mamma, d.h. auch in der Nähe des Brustkorbes, d.h. im Bereich der Behälteröffnung ermöglicht. Die Aufgabe wird dadurch gelöst, dass die Adapterauflage ein nachgiebiges ringförmiges Pufferelement (6) aufweist, das sich formschlüssig an das Objekt anlegt sowie das Pufferelement ein Dichtelement zwischen Objekt und Behälteröffnung bildet.

## Beschreibung

Die Erfindung betrifft eine Vorrichtung für eine Ultraschallgestützte Computertomographie vorzugsweise für Brustuntersuchungen (sonographische Mammographie) gemäß des ersten Patentanspruchs.

Ultraschalluntersuchungen gelten als bildgebende Diagnoseverfahren in der Medizintechnik nicht nur als besonders wirtschaftlich, sondern auch gewebeschonend.

Ein medizinisches Ultraschallgerät besteht im wesentlichen aus einem Schallkopf mit einer Anzahl von Ultraschallwandlern sowie einer Steuer- und Auswerteeinheit, welche die Steuerimpulse für die Ultraschallwandler aussendet sowie die im Patientenkörper reflektierenden Ultraschallsignale (Echosignale) wieder aufnimmt und als elektrische Signale verstärkt an die Auswerteeinheit weiterleitet (Impuls-Echo-Verfahren). Die Anzeige erfolgt in der Regel in Echtzeit an einem Bildschirm.

US 4.478.083 offenbart ein System für die Ultraschallmammographie, bei dem eine weibliche Brust von oben in einen zylindrischen Behälter eingeführt und positioniert wird. Zur Erfassung von Ultraschalleinzelaufnahmen weist der Behälter über der gesamten zylindrischen Wandungsfläche eine Vielzahl von unbeweglichen Ultraschallwandlern auf.

Ferner wird in DE 28 27 423 A1 eine Vorrichtung zur Ermittlung der inneren Struktur eines Körpers mit Hilfe von Schallstrahlen beschreiben, bei dem der Körper in ein mit einem Ankopplungsmedium gefüllten Behälter eingebracht und in diesem mit dem Ultraschalltransmissionsverfahren durchschallt wird. Dabei wird von einem oder mehreren Ultraschallsendern ein Schallstrahl durch den Körper auf mindestens einen Ultraschallwandler als Empfänger geschickt, die Empfangssignale in einer Auswerteeineinheit elektronisch weiterverarbeitet, gespeichert und anschließend die Verteilung des Schallbrechungsindexes sowie des Absorptionskoeffizienten ermittelt.

Auch die DE 100 50 232 A1 offenbart einen hochauflösenden Ultraschalltomograph für Gewebeuntersuchungen von Körperteilen und insbesondere der weiblichen Brust nach dem Transmissions-Streuungs- und Impuls-Echo-Verfahren. Er besteht aus einen oben offenen und mit einem Ankopplungsmedium gefüllten Behälter, in den der zu untersuchende Körperteil eingeführt wird, mit an der Behälterwandung über die gesamte Wandungsfläche fest angeordneten Ultraschallwandlern, deren Hauptabstrahlrichtung jeweils senkrecht von der Wandungsfläche in das Behälterinnere ausgerichtet ist. Eine rechnergestützte Steuer- und Auswerteeinheit dient der individuellen Ansteuerung von Ultraschallwandlern sowie der Visualisierung der von angewählten Empfängern empfangenen Signale.

Alle genannten Systeme ermöglichen eine hochauflösende tomographischen Abbildung einer in den Behälter von oben eingebrachten Extremität eines Patientenkörpers wie z.B. eine Mamma oder eines anderen beliebigen mit Ultraschallwellen durchdringbaren Körpers. Allerdings ist Benetzung eines Gewebebereichs mit Ankopplungsmedium nahe der oben liegenden Behälteröffnung problematisch und nur bedingt reproduzierbar. Die Anbindung der Ultraschallwellen ist in diesen Bereichen folglich nicht optimal, wodurch auch die Abbildungsgenauigkeit an ihre Grenzen stößt.

Bekanntlich entstehen Tumore in der Mamma zum überwiegenden Teil nicht in allzu weiter Entfernung zum Brustkorb, d.h. in einem Gewebebereich, der bei einer sonographischen Mammographie an einer der vorgenannten Ultraschallcomputertomographen nur wenig in den Behälter eintaucht und sich im Bereich der Behälteröffnung befindet. Eine Erfassung dieser Tumore mittels eines Ultraschallcomputertomographen der vorgenannten Art ist daher nur begrenzt und mit einer reduzierten Genauigkeit möglich. Davon ausgehend liegt die Aufgabe der Erfindung darin, eine Vorrichtung für eine Ultraschallgestützte Computertomographie mit einem erweiterten Messbereich vorzuschlagen, die insbesondere eine zuverlässige Mammographie der gesamten Mamma, d.h. auch in der Nähe des Brustkorbes, d.h. im Bereich der Behälteröffnung ermöglicht.

Die Aufgabe wird mit einer Vorrichtung mit den Merkmalen des Anspruch 1 gelöst. Unteransprüche geben vorteilhafte Ausführungsformen wieder.

Zur Lösung der Aufgabe wird eine Vorrichtung für eine Ultraschallgestützte Computertomographie an Objekten vorgeschlagen, umfassend einen mit einem Ankopplungsmedium gefüllten Behälter mit einer Behälteröffnung. Der Behälter weist eine Anzahl von Ultraschallwandlern auf, die im Behälter auf das Ankopplungsmedium einwirken. Dabei sind die Ultraschallwandler je nach Ausführungsform an der Wandung des Behälters oder in einem separaten im Behälter fernhantierbar beweglichen Träger wie z.B. ein Innenbehälter fixiert.

Ein Merkmal der Lösung umfasst eine Adapterauflage für ein Objekt, vorzugsweise die Patientenkörperoberfläche um eine zu untersuchende Extremität wie z.B. der Brustkorbbereich um eine zu untersuchende Brust einer Patientin auf der Behälteröffnung. Die Adapterauflage dient gleichzeitig als Dichtungselement zwischen Behälteröffnung und Objekt und verhindert auf diese Weise ein Austreten von Ultraschall-Ankopplungsmedium, vorzugsweise einem Gel (Ultraschallankopplungsgel) oder einer Flüssigkeit aus dem Behälter. Die Adapterauflage weist ein nachgiebiges Pufferelement auf, das sich ringförmig, formschlüssig und dichtend an das Objekt oder den vorgenannten Patientenkörper, vorzugsweise an den Brustkorb einer Patientin anlegt. Das Pufferelement wird vorzugsweise durch ein ringförmiges und mit einem Fluid oder Gel gefülltes Pufferelement gebildet. Es weist bevorzugt eine an das Objekt oder dem Patientenkörper angepasste Form vorzugsweise konfektioniert auf, wobei es je nach zu untersuchenden aufliegenden Objekt oder Patientenkörper in der Adapterauflage austauschbar gestaltbar ist. Das ringförmige Pufferelement ist folglich vorzugsweise nicht konstant dick und topograhisch und im Querschnitt gleich bleibend ausgeführt, sondern in ihrem Querschnitt dem jeweils zu überbrückenden Zwischenraum zwischen Patientenkörper oder Objekt und der Aufnahme des Pufferelements in der Adapterauflage überbrückend angepasst. Zur Vermeidung von ungewollten Ultraschallreflexionen am Pufferelement weist dieses eine akustische Impedanz auf, die vorzugsweise maximal 5% von der des Ankopplungsmediums abweicht. Vorzugsweise besteht das Pufferelement aus einer mit dem Ankopplungsmedium, vorzugsweise eine wässrige Lösung oder Wasser gefüllten Kunststoffhülle. Die Kunststoffhülle selbst ist bevorzugt reflexionsarm gegenüber Ultraschallwellen zu konzipieren, vorzugsweise dünn mit einer Wandstärke bevorzugt unter 1 mm, weiter bevorzugt unter 0,5 mm Wandstärke sowie mit rauen Oberflächen.

Die Adapterauflage, zumindest aber das Pufferelement ist optional austauschbar gestaltet. Sie begünstigt nicht nur eine schnelle Sterilisierbarkeit (oder Keimfreihaltung) der Ultraschallvorrichtung sondern auch eine Möglichkeit einer Anpassung der Vorrichtung an unterschiedliche Geometrien des Patientenkörpers oder des Objekts im Auflagebereich bei sonographischen Untersuchungen.

Vorzugsweise weist die Vorrichtung zusätzlich eine Ableitung für das Ankopplungsmedium im Bereich der Adapterauflage auf. Die Ableitung ist vorzugsweise als aktive Absaugung ausgeführt. Die Ableitung oder Absaugung findet vorzugsweise im Bereich der Behälteröffnung, d.h. im höchsten Punkt des für das Ankopplungsmedium im Behälter zur Verfügung stehenden Volumens statt. Damit wird auch sichergestellt, dass der Behälter bei aufliegendem Objekt oder Patienten vollständig mit Ankopplungsmedium gefüllt ist und insbesondere nach oben aufsteigende Luft oder Gasbestandteile abgeleitet oder abgesaugt werden.

Eine mögliche Ausführungsform weist eine Ableitung oder Absaugung der vorgenannten Art mit einer Ringdrainage in der Adapterauflage auf. Die Ringdrainage umfasst mindestens einen Ringkanal mit Abfluss und dient der Aufnahme von Leckströmen des Ankopplungsmediums, die zwischen Objekt oder Patienten und Pufferelement oder möglichen beweglichen (drehbaren oder verschiebbaren) Verbindungen zwischen Adapterauflage und Behälter auftreten. Der Ringkanal dient als Auffang- oder Sammelkanal für das aus dem Behälter austretende Ankopplungsmedium.

Eine weitere mögliche Ausführungsform umfasst Mittel für einen aktiven Fluidkreislauf für das Ankopplungsmedium. Ausgehend vom Behälterinneren erfolgt eine Weiterleitung des Ankopplungsmediums in die Ableitung oder Absaugung im Bereich der Behälteröffnung oder der Adapterauflage ggf. über einen weiteren Ringkanal oder einen anderen Sammel- oder Auffangkanal, und von dort in einen Abfluss oder Auslass. Der Abfluss oder Auslass mündet vorzugsweise direkt in eine Umwälzpumpe, vorzugsweise mit vorgeschalteten Gasabscheider und/oder Filter zur Abtrennung von gasförmigen bzw. festen Bestandteilen. Durch die Umwälzpumpe erfolgt eine Rückleitung des Ankopplungsmediums zurück in den Behälter.

Optional weist die Vorrichtung eine Verfahrvorrichtung des Behälters unterhalb der Adapterauflage auf. Eine vertikale Verfahrvorrichtung ermöglicht z.B. ein Anfahren des Behälters mit den Ultraschallwandlern von unten gegen eine auf der Adapterauflage aufliegende Patientin oder gegen einen aufliegenden Patienten oder ein Objekt. Laterale, d.h. horizontale Verstellmöglichkeiten oder Verdrehmöglichkeiten für ein oder mehrere translatorische bzw. rotatorische Freiheitsgrade ermöglichen zudem eine ggf. erforderliche Justierung der Ultraschallwandler zu dem zu untersuchenden Objekt. Zwischen den sich gegeneinander verschiebbaren Komponenten der Adapterauflage untereinander oder zum Behälterrand hin ist eine Abdichtung gegen ein Austreten von Ankopplungsmedium vorzusehen, z.B. eine Gleitdichtung oder eine Manschettenverbindung. Durch diese oder einem andere in Verbindung mit dem im Gehäuse integrierten Dichtelement wird die axial/radiale Bewegbarkeit des US-Messbehälters (Behälters) erheblich erweitert.

Durch die Adapterauflage als formschlüssige, an den Patientenkörper anschmiegsames Dichtelement sowie dem Behälter wird ein weitgehend dichtes Containment geschaffen, das mit Ankopplungsmedium wie z.B. Flüssigkeit gefüllt im Beispiel einer sonographischen Mammographievorrichtung praktisch die ganze Brust einschließt und sie somit einen erweiterten Bereich der Brust für die US-Mammographie zugänglich macht. Wesentlich bei der Erfindung ist ein größtmöglicher Zugangsbereich der Mamma oder ein anderes Objekt durch das Ultraschallankopplungsmedium und damit für die Ultraschallsignale. Insbesondere erweitert sich der Erfassungsbereich von sonographischen Untersuchungen mit Echoauswertung (Impuls-Echo-Verfahren) mit dem Zugangsbereich über die Mamma auch in die angrenzenden Bereiche der Patientin, d.h. über den Rand des Behälters hinaus in besonders vorteilhafter Weise, wenn vor allem eine ausreichende sonographische Anbindung im Bereich nahe der Adapterauflage und damit ein möglichst großer sonographischer Zugangswinkel zum zu untersuchenden Gewebe sichergestellt ist.

Eine vorgenannte dichtende Adapterauflage ermöglicht nicht nur eine maximal mögliche Vergrößerung der mit Ankopplungsmedium gefluteten Behälter-Kavitat bis zum Achselbereich einer Patientin, sondern auch die Verwendung besonders dünnflüssiger Ankopplungsmedien. Eine Ultraschall-Untersuchung ist bis über dem Rand des Messbehälters befindlichen Gewebes möglich, wobei eine Füllung des Pufferelements der Adapterauflage mit Ankopplungsmedium eine weitere Erweiterung des Messbereiches bewirkt.

Mögliche weitere Ausführungsformen der Erfindung werden anhand folgender Figuren näher erläutert. Es zeigen
**Fig.1** eine Schnittdarstellung einer Vorrichtung speziell für die Mammographie sowie
**Fig.2** die in **Fig.1** dargestellte Ausführung mit einer aufliegenden Patientin.

Die dargestellte Ausführungsform offenbart eine Vorrichtung für eine Ultraschallgestützte Computertomographie speziell an der weiblichen Brust zwecks sonographischer Mammographie mit erweitertem Messbereich. Die Figuren zeigen dabei die Vorrichtung ohne eine detaillierte Darstellung der dazugehörigen Einheiten für die Steuerung, Datenverarbeitung oder Visualisierung.

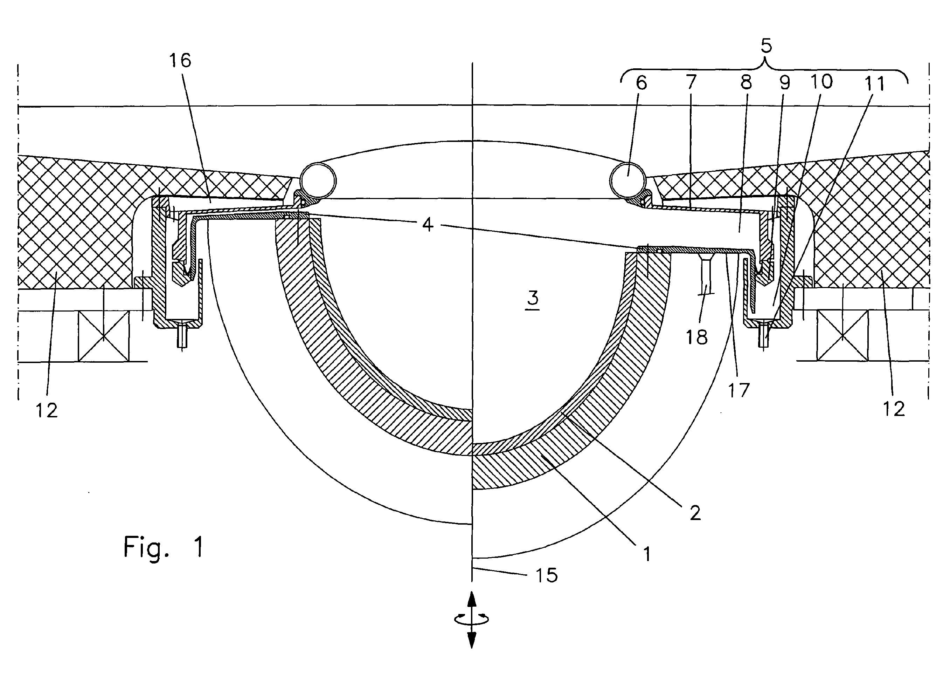

**Fig.1** zeigt die Vorrichtung ohne ein zu untersuchendes Objekt mit einem Behälter **1** einer Vielzahl in diesem innen anliegenden Ultraschallwandlern **2,** die in die Behälterkavität **3** ausgerichtet sind sowie einem nach oben hin offenen Behälterrand **4.** Der Behälter ist Schalen- oder Halbkugelförmig, d.h. er weist einen ausgerundeten und mit Ultraschallwandlern besetzten Boden auf. Damit eignet er sich besonders auch für die vorgenannte sonographische Erfassung von Körperregionen der Patientin, die oberhalb des Behälterrands **4** oder der auf diese aufgesetzte Adapterauflage **5** angeordnet sind, d.h. Bereich, die nicht von Ankopplungsmedium umspült werden.

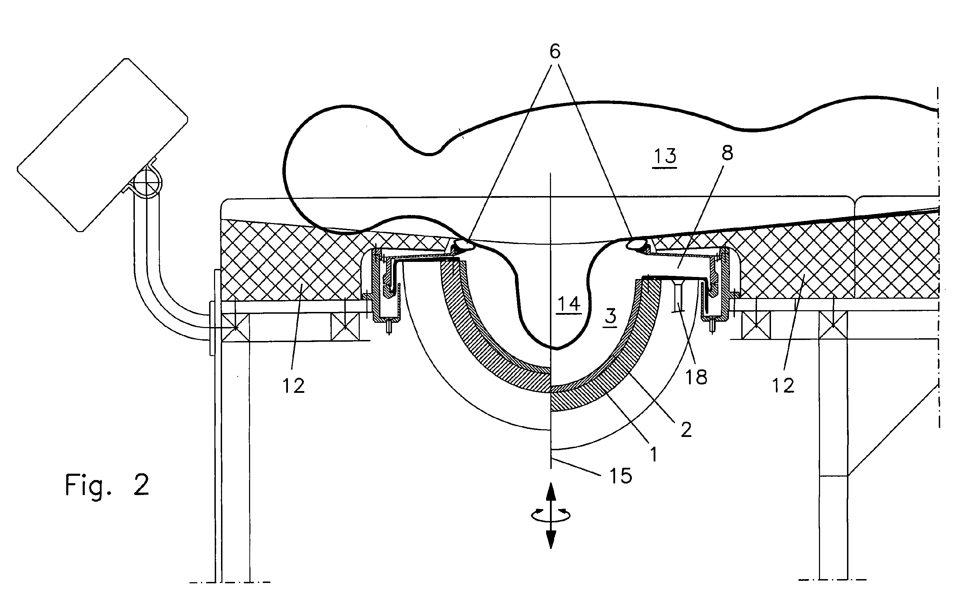

Die Adapterauflage **5** umfasst ein ringförmiges Pufferelement **6,** beispielhaft dargestellt mit einem kreisförmigen Querschnitt als eine dichtende formschlüssige Auflage einer Patientin sowie einem ringförmigen Träger **7** mit einem Ringkanal **8** mit Gleitdichtung **9** zu einer ringförmige Auffangwanne **10** mit Abfluss **11** für das aus dem Behälter in den Ringkanal als Auffangkanal ausmündende Ankopplungsmedium. Die Gleitdichtung wirkt gegen ein behälterseitiges Abschirmblech **17,** das zugleich die untere Begrenzung des Ringkanals **8** bildet. Sie ermöglicht vertikale und rotatorische Relativbewegungen von Behälter zu Adapterauflage. Die Auffangwanne **10** dient der Ableitung von Leckageverlusten des Ankopplungsmedium, das durch die genannte Gleitddichtung gedrückt wird oder zwischen Pufferlement **6** und Patientenkörper **13** (vgl. **Fig.2**) in einen Ablaufkanal **16** und von dort in die Auffangwanne **10** geleitet wird. Auffangwanne **10,** Abfluss **11** und Ablaufkanal **16** bilden somit eine Ringdrainage, wobei das hierdurch abgeleitete Ankopplungsmedium bevorzugt abgereinigt und / oder in einem Auffangbehälter zwischengelagert in den Behälter zurückgeführt wird.

Der Ringkanal **8** mündet in einen Auslass **18,** über den das Ankopplungsmedium abgelassen oder abgesaugt werden kann, bevorzugt im Rahmen eines vorgenannten Fluidkreislaufs. In diesen Fluidkreislauf wird vorzugsweise auch das über die Ringdrainage aufgefangene Ankopplungsmedium rückgespeist. Ein Auslass ist im Ausführungsbeispiel im Abschirmblech **17** eingesetzt. Diese Ausführung kommt ohne flexible Leitungen d.h. vorteilhaft nur mit starren Fluidverbindungen aus, wenn die Weiterleitung, Aufarbeitung und Wiedereinleitung des Ankopplungsmediums in den Behälter von mit dem Behälter fest verbundenen fluidischen Einheiten wie Pumpen, Filter oder Leitungen durchgeführt werden. Eine alternative Anordnung des Auslasses im Träger **7** begünstigt dagegen eine Weiterleitung und Aufarbeitung ohne flexible Rohrverbindungen in ortsfesten Systemen.

Über die Auffangwanne **10** ist der ringförmige Träger **7** und damit die Adapterauflage in einer Öffnung einer Patientenauflage **12** fixiert. Weitere eingangs genannte Ausgestaltungen der Erfindung sind mit der dargestellten Ausführung, wenn auch nicht dargestellt, kombinierbar.

**Fig.2** zeigt die in **Fig.1** dargestellte Ausführungsform mit einer auf der Patientenauflage aufliegenden Patientin **13** mit einer in die Behälterkavität **3** nach unten einhängenden Mamma **14.** Der Patientenkörper drückt dabei auf das Pufferelement **6,** drückt dieses etwas ein und geht mit diesem eine dichtende kraft- und formschlüssige Verbindung ein. Die für das Ankopplungsmedium zu Verfügung stehende Kavität im Behälter wird somit durch die Behälterwandungen oder Ultraschallwandler **2** einerseits und durch die Adapterauflage sowie die Mamma andererseits begrenzt.

**Fig.1** **und** **2** zeigen ferner eine Symmetrieachse **15** des Behälters. Die Figurhälfte links dieser Symmetrielinie zeigen jeweils einn Behälter in oberer, d.h. in der für eine sonographischen Untersuchung bevorzugten Position. Die rechten Bildhälften zeigen dagegen eine abweichende, d.h. vertikal abgesenkte Positionierung des Behälters mit einer entsprechenden Aufweitung des Ringspaltes **8.** Eine manuelle, hydraulische oder elektromechanische Verfahrvorrichtung für die vertikale Verstellung ist in den Figuren nicht angegeben, ist aber bevorzugt unterhalb des Behälters angeordnet und greift diesen bevorzugt im Bereich der Symmetrieaches **15** an. Alternative Verfahrvorrichtungen sind an der Adapterauflage angeordnet und greifen bevorzugt an mindestens drei am Umfang der Behälteröffnung angeordneten Stellen am Behälter an.

Die Ansteuerung der Ultraschallwandler **2** sowie einer Verstellvorrichtung ist nicht weiter dargestellt, erfolgt aber beispielsweise über eine Schnittstelle seitlich der Patientenliege, die einen schnellen Austausch der Vorrichtung z.B. an eine zentrale Hochleistungsrechnereinheit und damit eine schnelle Abfolge der sonographischen Untersuchungen einer Vielzahl von Patientinnen ermöglicht.

## Patentansprüche

1. Vorrichtung für eine Ultraschallgestützte Computertomographie an Objekten **(13)** mit erweitertem Messbereich, umfassend
a) einen mit einem Ankopplungsmedium gefüllten Behälter **(1)** mit einer Behälteröffnung,
b) eine Anzahl von Ultraschallwandlern **(2),** die in den Behälter auf das Ankopplungsmedium einwirken sowie
c) eine Adapterauflage **(5)** an der Behälteröffnung als Dichtelement zwischen Objekt und Behälteröffnung, **dadurch gekennzeichnet, dass**
d) die Adapterauflage ein nachgiebiges ringförmiges Pufferelement **(6)** aufweist, das sich formschlüssig an das Objekt anlegt sowie
e) das Pufferelement ein Dichtelement zwischen Objekt und Behälteröffnung bildet.

2. Vorrichtung nach Anspruch 1, **dadurch gekennzeichnet, dass** das Pufferelement **(6)** durch ein mit einem Fluid oder Gel gefülltes Pufferelement gebildet wird.

3. Vorrichtung nach Anspruch 2, **dadurch gekennzeichnet, dass** das Fluid oder Gel eine akustische Impedanz aufweist, die maximal 5% von der des Ankopplungsmediums abweicht.

4. Vorrichtung nach einem der vorgenannten Ansprüche, **dadurch gekennzeichnet, dass** die Adapterauflage oder das Pufferelement eine Zuleitung und/oder Ableitung **(8, 10, 11, 16, 18)** für das Ankopplungsmedium aufweist.

5. Vorrichtung nach Anspruch 4, **dadurch gekennzeichnet, dass** die Ableitung eine Ringdrainage **(10, 11, 16)** in der Adapterauflage **(5)** oder dem Pufferelement **(6)** umfasst.

6. Vorrichtung nach Anspruch 4 oder 5, **dadurch gekennzeichnet, dass** die Ringdrainage einen Überlauf über das Pufferelement **(6)** umfasst.

7. Vorrichtung nach einem der Ansprüche 4 bis 6, **dadurch gekennzeichnet, dass** Mittel für einen aktiven Fluidkreislauf für das Ankopplungsmedium vom Behälter **(1)** über die Ableitung **(8, 10, 11, 16, 18)**, einer Umwälzpumpe zurück in den Behälter vorgesehen ist.

8. Vorrichtung nach Anspruch 7, **dadurch gekennzeichnet, das** der Umwälzpumpe ein Gasabscheider und/oder ein Filter vorgeschaltet sind.

9. Vorrichtung nach einem der vorgenannten Ansprüche, **dadurch gekennzeichnet, dass** der Behälter **(1)** drehbar und/oder lateral verfahrbar gestaltet ist.

10. Vorrichtung nach Anspruch 9, **dadurch gekennzeichnet, dass** zwischen Adapterauflage **(5)** und Behälter **(1)** eine Gleitdichtung **(9)** vorgesehen ist.

11. Vorrichtung nach einem der vorgenannten Ansprüche, **dadurch gekennzeichnet, dass** das Pufferelement **(6)** austauschbar gestaltet ist. **Bezugszeichenliste**
1 Behälter
2 Ultraschallwandler
3 Behälterkavität
4 Behälterrand
5 Adapterauflage
6 Pufferelement
7 Träger
8 Ringkanal
9 Gleitdichtung
10 Auffangwanne
11 Abfluss
12 Patientenauflage
13 Patientin
14 Mamma
15 Symmetrieachse
16 Ablaufkanal
17 Abschirmblech
18 Auslass
